Europäisches Patentamt

European Patent Office

Office européen des brevets

(11) Veröffentlichungsnummer : **0 093 896**
**B1**

(12) **EUROPÄISCHE PATENTSCHRIFT**

(45) Veröffentlichungstag der Patentschrift :
10.09.86

(21) Anmeldenummer : 83103681.9

(22) Anmeldetag : 15.04.83

(51) Int. Cl.⁴ : **G 01 N 3/30, A 61 B 9/00**

(54) **Perkussionsinstrument.**

(30) Priorität : 26.04.82 DE 3215498

(43) Veröffentlichungstag der Anmeldung :
16.11.83 Patentblatt 83/46

(45) Bekanntmachung des Hinweises auf die Patenterteilung : 10.09.86 Patentblatt 86/37

(84) Benannte Vertragsstaaten :
AT CH DE FR GB IT LI SE

(56) Entgegenhaltungen :
DE-A- 2 617 779
US-A- 2 339 460
US-A- 4 289 023

(73) Patentinhaber : Siemens Aktiengesellschaft Berlin
und München
Wittelsbacherplatz 2
D-8000 München 2 (DE)

Fraunhofer-Gesellschaft zur Förderung der angewandten Forschung e.V.
Leonrodstrasse 54
D-8000 München 19 (DE)

Schulte, Willi, Prof. Dr.
Osianderstrasse 2 - 8
D-7400 Tübingen (DE)

Lukas, Dieter, Dipl.-Phys.
Osianderstrasse 2 - 8
D-7400 Tübingen (DE)

(72) Erfinder : Wohlgemuth, Jürgen
Brüder-Knauss-Strasse 61
D-6100 Darmstadt (DE)

(74) Vertreter : Mehl, Ernst, Dipl.-Ing. et al
Postfach 22 01 76
D-8000 München 22 (DE)

## Beschreibung

Die Erfindung bezieht sich auf ein Perkussionsinstrument mit einem beweglich gelagerten Stößel, der an seinem einen Ende einen Prüfkopf enthält und der mittels eines Antriebes aus einer Ausgangsstellung auf eine definierte Geschwindigkeit beschleunigt, danach, vom Antrieb abgekuppelt, mit gleichbleibender Geschwindigkeit im freien Flug auf ein Prüfobjekt zu bewegt und mit Hilfe eines Magnetfeldes wieder in seine Ausgangslage zurückgestellt wird.

Bei einem bekannten solchen Instrument (DE-OS-26 17 779) ist der Stößel in Achsrichtung des Instrumentes hin- und herbewegbar gelagert. Der Stößel wird durch eine im vorderen, den Prüfkopf enthaltenden Teil des Instrumentes angeordnete und mittels Magnetspule in der Ausgangsstellung gehaltene Spiralfeder auf eine bestimmte Geschwindigkeit beschleunigt. Nach vollständiger Entspannung der Feder löst sich der Stößel von der Feder und fliegt, in Lagern geführt, im freien Flug mit theoretisch konstanter Geschwindigkeit auf das zu prüfende Objekt zu. Nach Aufprall auf das Objekt wird durch den dabei entstehenden Gegenimpuls der Stößel wieder in Richtung Ausgangsstellung zurückgeworfen. Gegen Ende der Rückflugbewegung erhält die Spule einen Stromimpuls. Durch das dabei entstehende Magnetfeld wird der Stößel in seine Ausgangsstellung zurückgeholt und dabei die Feder erneut gespannt. Ein mit dem Stößel über ein biegeschlaff ausgebildetes Kabel verbundener Beschleunigungsaufnehmer erfaßt eine Geschwindigkeitsänderung beim Aufprall des Stößels auf das zu prüfende Objekt. Diese Geschwindigkeitsänderung kann während der Auslenk- und Rückstellbewegung für bestimmte Prüf- und Diagnosezwecke in einer mit dem Beschleunigungsaufnehmer verbundenen Auswerteelektronik ausgewertet werden ; in der Zahnheilkunde beispielsweise, um die Zahnbeweglichkeit bzw. den Lockerungsgrad eines Zahnes zu messen. Die Zeit, innerhalb der nach erfolgtem Impuls der zu prüfende Zahn wieder in seine Ausgangsstellung zurückkehrt, gibt z. B. Aufschluß über den Zustand des Zahnhalteapparates.

Bekannte Perkussionsinstrumente dieser Bauart sind mit verschiedenen Nachteilen behaftet. So wird der Stößel, der bei dem bekannten Instrument eine geradlinige, in Achsrichtung des Instruments verlaufende Flugbewegung ausführt und dabei eine konstante Geschwindigkeit beibehalten soll, von der Erdanziehungskraft beeinflußt. Weicht die Lage des Instrumentes von der Waagerechten ab, so verändert sich die Fluggeschwindigkeit des Stößels, wodurch die Meßergebnisse verfälscht werden. Um diesem vorzubeugen, müßte also das Instrument absolut waagerecht gehalten werden, was vom Anwender ein hohes Maß an Konzentration erfordern würde, die für diesen Zweck unangemessen wäre.

Ebenfalls von der Erdanziehungskraft beeinflußt ist die Vorrichtung gemäß US-A-23 39 460, bei der der Stößel als Langgestreckter Schwinghebel ausgebildet ist.

Ein weiterer Nachteil ist darin zu sehen, daß die Prüfobjekte, z. B. die Zähne, mit dem bekannten Instrument nur von der Seite aus angestoßen werden können, von der das Instrument in der waagerechten Haltung dem Objekt zuführbar ist.

Prüfobjekte, die in der Richtung, in der das Instrument an das zu prüfende Objekt zugeführt wird, nicht zugänglich sind, können deshalb nicht bzw. nicht zuverlässig gemessen werden. Bei Anwendung des Instruments zur Überprüfung der Zahnbeweglichkeit sind deshalb die im molaren Bereich liegenden Zähne mit dem bekannten Perkussionsinstrument nicht bzw. nur schlecht zu erfassen.

Weitere Nachteile sind in der Anordnung des Beschleunigungsaufnehmers und der damit zu gewinnenden Beschleunigungssignale zu sehen. Der bei dem bekannten Instrument vorgesehene Beschleunigungsaufnehmer ist an dem dem Prüfkopf abgewandten Ende des Stößels angeordnet und über eine hochflexible Zufuhrleitung mit der Auswerteelektronik verbunden. Nachdem die Zuleitung praktisch die gesamte Stößelbewegung mitmachen bzw. ausgleichen muß, kann es zu einem vorzeitigen Verschleiß (Bruch) der Leitung kommen. Das mit dem Beschleunigungsaufnehmer der bekannten Bauart gewonnene Beschleunigungssignal ist außerdem relativ klein und muß durch aufwendige Schaltungsmaßnahmen verstärkt werden. Ein weiterer Nachteil ist darin zu sehen, daß der in Gleitlagern gelagerte Stößel von den Reibungskräften im Lager gebremst wird. Diese Reibungskräfte können sich durch äußere Einflüsse stark verändern, z. B. durch Feuchtigkeit, Staub usw. Die Stößelgeschwindigkeit ist dadurch gewissen Veränderungen unterworfen, die ebenfalls die Meßergebnisse verfälschen.

Zur Beseitigung der den bekannten Perkussionsinstrumenten anhaftenden Nachteile wird erfindungsgemäß vorgeschlagen, den Stößel als langgestreckten Schwinghebel auszubilden, der in seinem Schwerpunkt um eine quer zur Längsachse des Instrumentes liegende Achse bewegbar gelagert ist, den Prüfkopf winkelig, vorzugsweise rechtwinkelig, zur Längsachse des Schwinghebels auszubilden und am Prüfkopf einen an sich bekannten Beschleunigungsaufnehmer anzubringen.

Das vorgeschlagene Perkussionsinstrument arbeitet lageunabhängig und unbeeinflußt von der Schwerkraft.

Durch die winkelige Anordnung des Prüfkopfes sind Messungen auch an relativ schlecht zugänglichen Stellen, z. B. im molaren Bereich der Zähne, möglich.

Vorteilhafte Weiterbildungen und Ausgestaltungen der Erfindung sind in den Unteransprüchen enthalten.

Die Anordnung eines Beschleunigungsaufnehmers im vorderen, den Prüfkopf enthaltenden Abschnitt des Stößels hat den Vorteil, daß man ein erheblich größeres Beschleunigungssignal beim Stoß auf das zu prüfende Objekt und ein geringeres Störsignal bei der Rückwärtsbewegung erhält als bisher. Nachdem der Stößel praktisch reibungsfrei im Schwerpunkt gelagert ist, ist die Lagerreibung bei der Bewegung des Stößels praktisch vernachlässigbar. Durch die vorgeschlagene Anordnung ist es auch möglich, bei Verwendung eines Beschleunigungsaufnehmers das erforderliche Zuleitungskabel so zu führen, daß dieses bei der Schwingbewegung des Stößels praktisch nicht belastet wird.

Ausführungsbeispiele der Erfindung werden nachfolgend anhand der Zeichnung näher erläutert. Es zeigen :

Figur 1 ein Perkussionsinstrument nach der Erfindung in einer schaubildlichen Darstellung,

Figur 2 einen Teil des Instrumentes nach Fig. 1 in einer Draufsicht,

Figur 3 das Instrument nach Fig. 1 im Längsschnitt,

Figur 4 einen Teil des Schwinghebels im Längsschnitt,

Figur 5 eine Ausführungsform einer Schaltungsanordnung zur Ansteuerung des Antriebs,

Figur 6 eine andere Ausführungsform des Instrumentes im Längsschnitt.

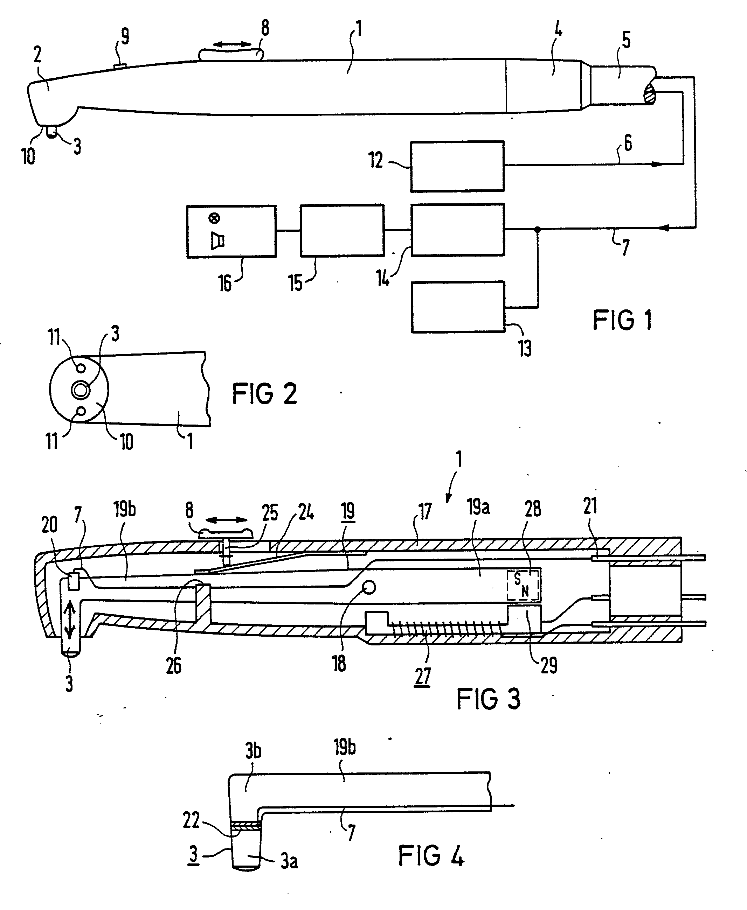

Die Fig. 1 zeigt in einer Seitenansicht ein Perkussionsinstrument mit einem länglichen Handgriff 1, der an seinem vorderen, abgewinkelten Ende in ein Kopfgehäuse 2 übergeht, aus dem stirnseitig ein Prüfkopf 3 eines in den nachfolgenden Figuren noch näher ersichtlichen Schwinghebels vorsteht. An dem dem Kopfgehäuse 2 abgewandten Ende des Handgriffes 1 ist mittels einer Anschlußarmatur 4 ein Versorgungsschlauch 5 angeschlossen, der Zuleitungen 6, 7 für eine noch näher erläuterte Schaltungsanordnung enthält.

Mit 8 ist ein von Hand betätigbarer Schieber, mit 9 eine Anzeigeleuchte bezeichnet.

Die Stirnseite 10 des Kopfgehäuses 2 enthält am Umfang zwei Lichtquellen 11 (Fig. 2), die so fokussiert sind, daß sie am Prüfobjekt (Zahn) gebündeltes Licht liefern, und zwar in Form eines kreisrunden Lichtfleckes in Ebenen quer zur Längsachse des Prüfkopfes 3, also parallel zur Stirnfläche 10 des Kopfgehäuses 2.

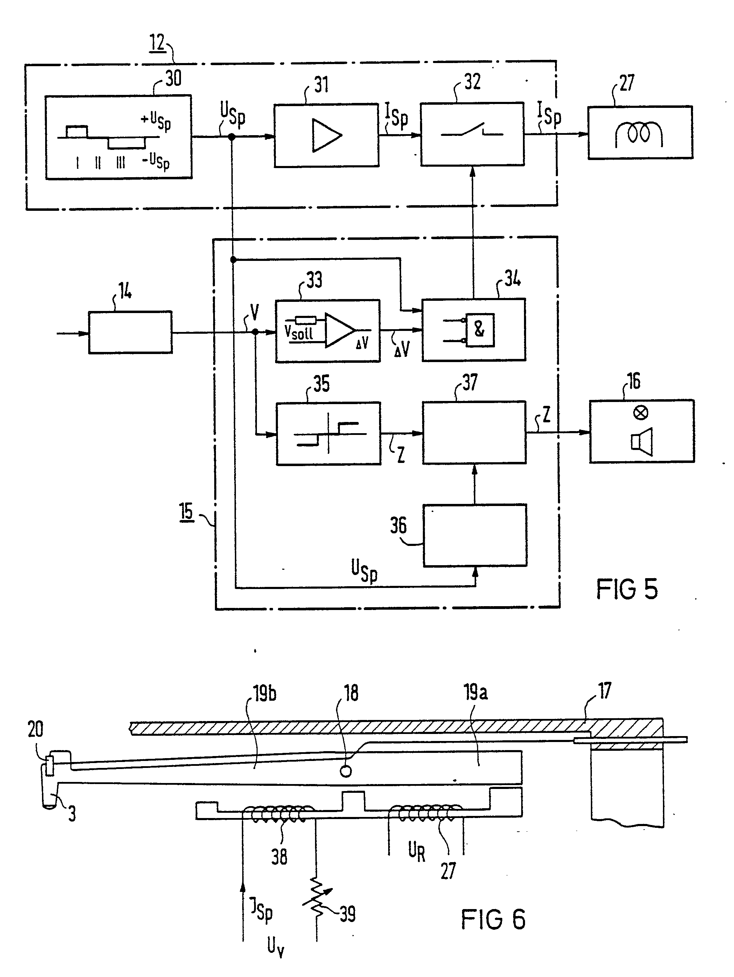

Die Zuleitung 6 ist mit einer Steuerelektronik 12 verbunden, die in Fig. 5 noch näher aufgezeigt ist und einem im Inneren des Handgriffes 1 angeordneten Antrieb periodisch Stromimpulse zuführt.

Über die Leitung 7 werden die vom Prüfkopf 3 bei dessen Anschlag am zu prüfenden Objekt erhaltenen Beschleunigungssignale einerseits einer Auswerteelektronik 13 zur weiteren objektbezogenen Auswertung zugeführt, andererseits in einer Integrierstufe 14 nach der Beziehung $v = \int b\, dt$ in Geschwindigkeitssignale umgewandelt, die in einer Meß- und Auswerteeinrichtung 15 weiterverarbeitet werden. In einer Anzeigeeinrichtung 16 kann dem Anwender eine akustische oder optische Information gegeben werden.

Die Fig. 3 zeigt das Instrument nach Fig. 1 im Längsschnitt. Im Gehäuse 17 des Handgriffes 1 ist mittels einer quer zur Längsachse des Instrumentes verlaufenden Achslagerung 18 ein langgestreckter, den Prüfkopf 3 enthaltender Schwinghebel 19 bewegbar gelagert. Die Lagerung erfolgt im Schwerpunkt des Schwinghebels mittels nahezu reibungsfreier Lager (Spitzenlager oder Kugellager). Die Lagerung unterteilt den Schwinghebel 19 in einen rückwärtigen Abschnitt 19a und einen vorderen, den Prüfkopf 3 aufnehmenden Abschnitt 19b. Der Prüfkopf 3 ist in bezug auf den Schwinghebel 19 nahezu um 90° abgewinkelt und an seinem vorderen Ende ballig ausgebildet. Der Schwinghebelabschnitt 19b ist im Vergleich zum rückwärtigen Abschnitt 19a relativ lang ausgebildet, wodurch der Prüfkopf 3 im Bereich des zur Anwendung erforderlichen Hubes von etwa 2 bis 8 mm eine nahezu geradlinige Bewegung in Pfeilrichtung beschreibt.

Am Prüfkopf 3, also in unmittelbarer Nähe des zu prüfenden Objektes, ist ein Beschleunigungsaufnehmer 20 befestigt ; dessen Zuleitung 7 ist bis zur Achslagerung 18 im oder am Schwinghebel 19 befestigt und in unmittelbarer Nähe der Lagerachse 18 aus dem beweglichen Schwinghebel heraus zu einem am Gehäuse 17 angeordneten Anschlußglied 21 geführt. Die Anordnung des Beschleunigungsaufnehmers 20 in unmittelbarer Nähe des Prüfobjektes hat den Vorteil, daß, nachdem hier die größte Bewegung stattfindet, das höchste Beschleunigungssignal abgegriffen werden kann. Der Beschleunigungsaufnehmer kann deshalb sehr klein ausgebildet sein.

Eine besonders vorteilhafte Anordnung und Ausbildung eines Beschleunigungsaufnehmers ist in Fig. 4 dargestellt. Der Beschleunigungsaufnehmer besteht hier aus zwei übereinander angeordneten piezokeramischen Elementen 22, die den Prüfkopf 3 in einen die Prüfspitze enthaltenden Abschnitt 3a mit relativ kleiner Masse und einen Abschnitt 3b, der in den Schwinghebelabschnitt 19b übergeht, mit im Vergleich zum Abschnitt 3a erheblich größerer Masse, unterteilen. Bei Aufprall des Prüfkopfes 3 auf das zu prüfende Objekt wird nahezu die gesamte Masse des Schwinghebels als Trägheitskraft auf die piezoelektrischen Elemente wirksam. Dadurch kann ein um mehrere Zehnerpotenzen erhöhtes Nutzsignal über die Zuleitung 7 abgenommen werden. Bei Rückführung des Schwinghebels in die Ausgangsposition und bei Anschlag an die dort befindlichen Begrenzungen wirkt infolge der relativ kleinen Masse des Abschnittes 3a, welche auf die Piezoelemente wirken, dagegen eine geringe Trägheitskraft auf die Elemente, wodurch nur ein geringfügiges Störsignal erzeugt wird.

Als Antrieb für die Vorwärtsbewegung des Schwinghebels 19 ist eine im Gehäuse 17 befestigte Blattfeder 24 vorgesehen, die mittels eines am Schieber 8 befestigten Druckstiftes 25 gegen den Schwinghebelabschnitt 19b drückt und so den Schwinghebel aus der gezeichneten Ruhestellung heraus auf die erforderliche Geschwindigkeit hin beschleunigt. Ist die Geschwindigkeit erreicht, wird die Blattfeder 24 von einem seitlich des Schwinghebels am Gehäuse angeordneten Anschlag 26 blockiert, wodurch diese vom Schwinghebel abgekoppelt wird. Der Schwinghebel bewegt sich danach kräftefrei und mit konstanter Geschwindigkeit im freien Flug weiter bis er am Prüfobjekt oder gegen einen Endanschlag anschlägt.

Die Rückführung des Schwinghebels 19 in seine Ausgangsposition erfolgt mittels eines Elektromagneten 27, der vorteilhafterweise korrespondierend dem Schwinghebelabschnitt 19a angeordnet ist und mittels einer Steuerelektronik mit Stromimpulsen angesteuert wird.

Mittels des Schiebers 8 kann die Blattfeder 24 mehr oder weniger vorgespannt und damit die Geschwindigkeit des Schwinghebels für die Vorwärtsbewegung beeinflußt werden.

Der vorgenannte Antrieb ist äußerst einfach und erfordert praktisch keinen größeren Steuerungsaufwand. Denkbar ist es aber auch, den Antrieb für die Vorwärtsbewegung elektromagnetisch auszuführen. Gemäß einer vorteilhaften Ausgestaltung der Erfindung wird demnach vorgeschlagen, am Ende des Schwinghebelabschnittes 19a einen Permanentmagneten 28 anzuordnen, der mit dem Ende 29 des Elektromagneten so korrespondiert, daß je nach Richtung des über die Steuerelektronik 12 gesteuerten Spulenstroms eine anziehende bzw. abstoßende Wirkung eintritt.

Die Ansteuerung der Spule 27, die bei dieser Lösung sowohl für die Vor- als auch für die Rückwärtsbewegung des Schwinghebels 19 dient, sei anhand der Blockschaltbilder nach Fig. 1 und 5 näher erläutert.

Die Steuerelektronik 12 enthält einen Impulsgeber 30, der für eine Vorwärtsbewegung des Stößels an die Antriebsspule 27 ein positives Signal (+ $U_{SP}$) und für die Rückwärtsbewegung ein negatives Signal (− $U_{SP}$) liefert. Zeitlich zwischen den Phasen der Vorwärtsbewegung (I) und der Rückwärtsbewegung (III) liegt die Freiflugphase (II), in der der Schwinghebel 19 ohne zusätzlichen Antrieb nach vorne gedrückt wird. Die nachfolgende Verstärkerstufe 31 mit dem Schalter 32 schaltet entsprechend den Strom für die Antriebsspule 27. Der Schalter 32 unterbricht dabei den Antriebsstrom während der Vorwärtsbewegung (Takt I des Impulsgebers 30), wenn der Schwinghebel 19- seine Sollgeschwindigkeit erreicht hat. Dazu wird das aus der Integrierstufe 14 gewonnene Geschwindigkeitssignal in einem Soll-Istwertvergleicher 33 mit einem vorgegebenen Sollwert verglichen. Ein Schaltbefehlgeber 34 öffnet den Schalter 32, wenn die Geschwindigkeitsdifferenz (Δ v) Null und die Impulsgeberspannung größer als Null ist.

Das Geschwindigkeitssignal v wird gleichzeitig einem Grenzwertmelder 35 zugeführt, der ein Überschreiten der zulässigen Abweichungen von der Sollgeschwindigkeit während der Freiflugphase des Schwinghebels (Takt II des Impulsgebers 30) mißt und in diesem Fall über die Anzeigeeinheit (Signalgeber) 16, die z. B. in Form der Anzeigelampe 9 (Fig. 1) am Handgriff angebracht sein kann, dem Benutzer des Handstückes optisch oder akustisch auf eine nicht zulässige Abweichung hinweist.

Ein Schaltbefehlgeber 36 sorgt dafür, daß das Fehlersignal vom Schalter 37 nur dann durchgeschaltet wird, wenn sich der Schwinghebel im Freiflug (Takt II des Impulsgebers) befindet. In diesem Fall ist die anliegende Spannung $U_{SP}$ = 0.

Die Fig. 6 zeigt eine Ausführungsform, bei der die Vorwärtsbewegung ebenfalls elektromagnetisch erfolgt, und zwar mittels eines Elektromagneten 38, der dem Schwinghebelabschnitt 19b korrespondierend angeordnet ist. Auch hier besteht zumindest der mit dem Elektromagneten 38 korrespondierende Teil des Schwinghebels aus weichmagnetischem Material. Die Steuerung des Spulenstroms für die beiden Elektromagnete 27, 38 erfolgt über einen Impulsgeber, der die beiden Elektromagneten wechselweise mit Stromimpulsen versorgt, wodurch der Schwinghebel die bereits erläuterte hin- und herschwingende Bewegung ausführt. Die Bewegungsgeschwindigkeit in Stoßrichtung kann durch ein elektrisches Stellglied 39, welches im Stromkreis der Antriebsspule 38 eingeschaltet ist, beeinflußt werden.

Wie eingangs in Betrachtung der Fig. 2 bereits angesprochen, können, um die Anwendung des Instrumentes für den Benutzer noch weiter zu vereinfachen, in Nähe des Prüfkopfes 3 im oder am Gehäuse ein oder mehrere Lichtquellen in Form von Miniaturglühlampen oder eines Lichtleiters vorgesehen sein.

Die Lichtquellen sind so angeordnet, daß der austretende Lichtstrahl scharf gebündelt ist und auf dem Prüfobjekt dann, wenn die Stirnfläche 10 (Fig. 1) des Instruments parallel zum Objekt verläuft, als runder Lichtfleck erscheint. Der Anwender kann so erkennen, ob er das Instrument so hält, daß die Stoßbewegung senkrecht zur Zahnachse erfolgt. Bei Abweichungen von der Senkrechten wird der Lichtfleck elliptisch, wodurch dem Anwender angezeigt wird, daß er seine Instrumentenhaltung zu korrigieren hat.

**Patentansprüche**

1. Perkussionsinstrument mit einem beweglich gelagerten Stößel, der an seinem einen Ende einen Prüfkopf (3) enthält und der mittels eines Antriebes aus einer Ausgangsstellung auf eine definierte Geschwindigkeit beschleunigt, danach vom Antrieb abgekuppelt, mit gleichbleibender Geschwindigkeit im freien Flug auf ein Prüfobjekt zu bewegt und mit Hilfe eines Magnetfeldes wieder in seine Ausgangsposition zurückgestellt wird, dadurch gekennzeichnet, daß der Stößel als langgestreckter Schwinghebel (19) ausgebildet ist, der in seinem Schwerpunkt um eine quer zur Längsachse des Instrmentes liegende Achse (18) bewegbar gelagert ist, daß der Prüfkopf (3) winkelig, vorzugsweise rechtwinkelig, zur Längsachse des Schwinghebels (19) angeordnet ist und daß am Prüfkopf (3) ein an sich bekannter Beschleunigungsaufnehmer (20, 22) angebracht ist.

2. Perkussionsinstrument nach Anspruch 1, dadurch gekennzeichnet, daß von den durch die Achslagerung (18) unterteilten Abschnitten (19a, 19b) des Schwinghebels (1) der den Prüfkopf (3) aufnehmende Abschnitt (19b) relativ lang ausgebildet ist.

3. Perkussionsinstrument nach Anspruch 1 oder 2, dadurch gekennzeichnet, daß Zuleitungen (7) des Beschleunigungsaufnehmers (20, 22) im oder am Schwinghebel (19) entlang geführt und im Bereich der Achslagerung (18) des Schwinghebels aus diesem herausgeführt sind.

4. Perkussionsinstrument nach einem der Ansprüche 1 bis 3, dadurch gekennzeichnet, daß als Antrieb für die Vorwärtsbewegung des Schwinghebels (19) eine im vorderen, den Prüfkopf (3) aufnehmenden Abschnitt (19b) angeordnete Feder (24) vorgesehen ist, die den Schwinghebel aus der Ruhestellung heraus auf die erforderliche Geschwindigkeit beschleunigt, und daß Anschlagmittel (26) vorhanden sind, die in einer bestimmten Bewegungsphase des Schwinghebels die Feder (24) blockieren und damit den Antrieb vom Schwinghebel abkoppeln.

5. Perkussionsinstrument nach Anspruch 4, dadurch gekennzeichnet, daß die Antriebsfeder (24) mittels eines mechanischen Stellgliedes (8, 25) in ihrer Spannkraft eingestellt werden kann.

6. Perkussionsinstrument nach Anspruch 5, dadurch gekennzeichnet, daß als Antriebsfeder eine Blattfeder (24) und als Stellglied ein auf die Blattfeder drückender, mittels eines Schiebers (8) längs des Instrumentes verstellbarer Druckstift (25) vorgesehen ist.

7. Perkussionsinstrument nach einem der Ansprüche 1 bis 3, dadurch gekennzeichnet, daß als Antrieb für die Vor- und Rückwärtsbewegung des Schwinghebels ein magnetischer Antrieb, vorzugsweise ein elektromagnetischer Antrieb (27, 28 ; 27, 38), vorgesehen ist.

8. Perkussionsinstrument nach Anspruch 7, dadurch gekennzeichnet, daß beide Abschnitte (19a, 19b) des Schwinghebels (19) zumindest teilweise aus weichmagnetischem Material bestehen, und diesen weichmagnetischen Abschnitten (19a, 19b) Magnetspulen (27, 38) zugeordnet sind, welche im nichtbeweglichen Teil (17) des Instruments angeordnet sind und von einer Steuerelektronik (12) wechselweise mit Impulsen angesteuert werden.

9. Perkussionsinstrument nach Anspruch 8, dadurch gekennzeichnet, daß in den Stromkreis der Antriebsspule (38) ein die Antriebskraft veränderndes Stellglied (39) eingeschaltet ist.

10. Perkussionsinstrument nach einem der Ansprüche 1 bis 3, dadurch gekennzeichnet, daß der dem Prüfkopf (3) abgewandte Abschnitt (19a) des Schwinghebels (19) zumindest teilweise aus weichmagnetischem Material besteht und diesem Abschnitt (19a) korrespondierend im Instrumentengehäuse (17) ein Elektromagnet (27) angeordnet ist, daß im Bereich des Magnetfeldes des zum Antrieb dienenden Elektromagneten (27), vorzugsweise an dem dem Prüfkopf (3) abgewandten Ende, ein Permanentmagnet (28) derart angeordnet ist, daß je nach Richtung des Spulenstroms ($J_{sp}$) eine anziehende bzw. abstoßende Wirkung erzeugt wird.

11. Perkussionsinstrument nach einem der Ansprüche 1 bis 10, dadurch gekennzeichnet, daß an der Austrittsstelle des Prüfkopfes (3) im Instrumentengehäuse (17) eine Beleuchtungseinrichtung (11) vorgesehen ist, die einen scharf gebündelten Lichtfleck erzeugt, der in Ebenen senkrecht zur Bewegungsrichtung des Prüfkopfes (3) kreisrund ist.

12. Perkussionsinstrument nach einem der Ansprüche 1 bis 11, dadurch gekennzeichnet, daß der Schwinghebel (19) in der Beschleunigungsphase vor Auftreffen auf das Objekt von einem Anschlag (26) abgefangen wird und das dadurch im Beschleunigungsaufnehmer (20) erzeugte Signal einem Verstärker (31) zugeführt wird und danach in einem Soll-Istwertvergleicher (33) verglichen wird, dergestalt, daß bei Abweichen vom Sollwert ein optisches oder akustisches Signal erzeugt wird.

13. Perkussionsinstrument nach Anspruch 12, dadurch gekennzeichnet, daß am Instrument eine Anzeigeleuchte (9) angeordnet ist.

14. Perkussionsinstrument nach einem der Ansprüche 1 bis 13, dadurch gekennzeichnet, daß als Beschleunigungsaufnehmer ein oder mehrere, an sich bekannte piezokeramische Elemente (22) vorgesehen sind, die im vorderen Teil des den Prüfkopf (3) aufnehmenden Abschnittes (19b) des Schwinghebels (19), vorzugsweise im Prüfkopf (3) selbst, so angeordnet sind, daß bei Aufprall des Prüfkopfes (3) auf das Prüfobjekt ein Großteil der Masse des Schwinghebels (19) als Trägheitskraft auf die Elemente (22) wirkt.

**Claims**

1. A percussion apparatus having a movably positioned tappet, one end of which comprises a testing head (3) and by means of a drive is accelerated from an initial position to a defined speed, subsequently decoupled from the drive, moved in free flight to an object to be tested at a constant speed, and returned into its initial position with the aid of a magnetic field, characterised in that the tappet is designed as an elongate rocking lever (19) pivotably mounted at its centre of gravity about an axle (18) arranged transversely to the longitudinal axis of the apparatus, that the testing head (3) is arranged at an angle, preferably at right angles, to the longitudinal axis of the rocking lever (19) and that an acceleration pickup (20, 22), known *per se*, is arranged on the testing head (3).

2. A percussion apparatus as claimed in Claim 1, characterised in that the rocking lever (1) is divided into sections (19a, 19b) by the axle bearing (18), and the section (19b) which accommodates the testing head (3) is constructed so as to be relatively long.

3. A percussion apparatus as claimed in Claim 1 or 2, characterised in that supply lines (7) of the acceleration pickup (20, 22) are guided in or along the rocking lever (19) and guided away from the rocking lever in the region of the axle bearing (18) thereof.

4. A percussion apparatus as claimed in one of Claims 1 to 3, characterised in that a spring (24) is arranged in the front section (19b) which accommodates the testing head (3) as a drive for the forward motion of the rocking lever (19), which spring accelerates the rocking lever from the rest position to the required speed, and that stop means (26) are provided which block the spring (24) in a specific phase of motion of the rocking lever and thus decouple the drive from the rocking lever.

5. A percussion apparatus as claimed in Claim 4, characterised in that the drive spring (24) tensional force can be adjusted by means of a mechanical adjusting element (8, 25).

6. A percussion apparatus as claimed in Claim 5, characterised in that a flat spring (24) is provided as a drive spring and a pressure pin (25) which presses onto the flat spring and is adjustable along the apparatus by means of a slide (8) is provided as an adjusting element.

7. A percussion apparatus as claimed in one of Claims 1 to 3, characterised in that a magnetic drive, preferably an electro-magnetic drive (27, 28 ; 27, 38) is provided as a drive for the forward and backward motion of the rocking lever.

8. A percussion apparatus as claimed in Claim 7, characterised in that both sections (19a, 19b) of the rocking lever (19) at least partially consist of a soft-magnetic material and the soft-magnetic sections (19a, 19b) are assigned magnetic coils (27, 38) arranged in the non-movable component (17) of the apparatus and alternately controlled by a control electronics unit (12) by means of pulses.

9. A percussion apparatus as claimed in Claim 8, characterised in that a control component (39) which changes the drive force is connected into the circuit of the drive coil (38).

10. A percussion apparatus as claimed in one of Claims 1 to 3, characterised in that the section (19a) of the rocking lever (19) remote from the testing head (3) at least partially consists of a soft-magnetic material and the section (19a) is correspondingly assigned an electro-magnet (27) in the case (17) of the apparatus, and in the region of the magnetic field of the electro-magnet (27) which serves as a drive, preferably at the end remote from the testing head (3), there is arranged a permanent magnet (28) in such a manner that depending upon the direction of the coil current ($J_{sp}$) an attractive or repulsive effect is produced.

11. A percussion apparatus as claimed in one of Claims 1 to 10, characterised in that at the outlet point of the testing head (3) in the case (17) of the apparatus an illuminating device (11) is arranged which produces a sharply focussed light spot which is circular in planes at right angles to the direction of movement of the testing head (3).

12. A percussion apparatus as claimed in one of Claims 1 to 11, characterised in that during the acceleration phase, prior to hitting the object, the rocking lever (19) is supported by a stop (26) and the signal which is thereby produced in the acceleration pickup (20) is fed to an amplifier (31) and subsequently compared in a theoretical/actual value comparator (31) in such a manner that in the event of a deviation from the theoretical value an optical or acoustic signal is generated.

13. A percussion apparatus as claimed in Claim 12, characterised in that an indicator lamp (9) is arranged on the apparatus.

14. A percussion apparatus as claimed in one of Claims 1 to 13, characterised in that one or more piezo-ceramic elements (22) known *per se* are provided as an acceleration pickup, which elements are arranged in the front part of the section (19b) of the rocking lever (19), which accommodates the testing head (3), preferably in the testing head (3) itself, in such manner that upon impact of the testing head (3) onto the object to be tested, a large part of the mass of the rocking lever (19) has the effect of an inertia force on the elements (22).

**Revendications**

1. Instrument de percussion avec un poussoir monté avec mobilité, lequel poussoir comporte à l'une de ses extrémités, une tête d'examen (3) et est accéléré, à l'aide d'un dispositif d'entraînement, à partir d'une position de départ à une vitesse définie pour être ensuite, en étant détaché du dispositif d'entraînement, déplacé librement et avec une vitesse constante, sur un objet à examiner et ramené, à l'aide d'un champ magnétique, dans sa position de départ, caractérisé par le fait que le poussoir est réalisé sous la forme d'un levier oscillant allongé (19) qui est monté avec mobilité, en son centre de gravité, sur un axe (18) situé transversalement par rapport à l'axe longitudinal de l'instrument, que la tête d'examen est disposée de manière à former un angle, de préférence un angle droit avec l'axe longitudinal du levier oscillant (19) et que sur la tête d'examen (3) est monté un capteur d'accélération (20, 22) connu en soi.

2. Instrument de percussion selon la revendication 1, caractérisé par le fait que parmi les sections (19a, 19b) du levier oscillant, qui sont subdivisées par le montage sur l'axe (18), la section (19b) qui reçoit la tête d'examen (3) est réalisée avec une longueur relativement importante.

3. Instrument de percussion selon la revendication 1 ou 2, caractérisé par le fait que des conducteurs d'amenée (7) du capteur d'accélération (20, 22) sont guidés dans ou le long du levier oscillant (19) et sortent de celui-ci dans la zone du montage sur l'axe de montage (18).

4. Instrument de percussion selon l'une des revendications 1 à 3, caractérisé par le fait que l'on prévoit, en tant que dispositif d'entraînement pour le mouvement avant du levier oscillant (19), un ressort (24) disposé dans la section antérieure (19b) qui reçoit la tête d'examen (3), ledit ressort (24) accélérant le levier oscillant à partir de sa position de repos à la vitesse nécessaire, et que des moyens de butée (26) sont prévus, qui, dans une phase déterminée du mouvement du levier oscillant, bloquent le ressort (24) et séparent le dispositif d'entraînement du levier oscillant.

5. Instrument de percussion selon la revendication 4, caractérisé par le fait que le ressort d'entraînement peut être réglé, du point de vue de sa force élastique, à l'aide d'un organe mécanique de réglage (8, 25).

6. Instrument de percussion selon la revendication 5, caractérisé par le fait qu'il est prévu, en tant que ressort d'entraînement, un ressort à lame (24) et comme organe de réglage une cheville de pression qui appuie sur le ressort à lame et qui est réglable à l'aide d'un coulisseau (8) le long de l'instrument.

7. Instrument de percussion selon l'une des revendications 1 à 4, caractérisé par le fait qu'il est prévu, en tant que dispositif d'entraînement pour le mouvement avant et arrière du levier oscillant, un entraînement magnétique, de préférence un entraînement électromagnétique (27, 28 ; 27, 38).

8. Instrument de percussion selon la revendication 6, caractérisé par le fait que les deux sections (19a, 19b) du levier oscillant (19) sont constituées, au moins partiellement, par un matériau magnétique doux et qu'à ces sections en matériau magnétiquement doux (19a, 19b) sont associés des électro-aimants (27, 38) qui sont disposés dans la partie immobile (17) de l'instrument et qui sont attaqués alternativement par des impulsions à l'aide d'une électronique de commande (12).

9. Instrument de percussion selon la revendication 8, caractérisé par le fait que l'on prévoit dans le circuit de la bobine d'entraînement (38) un organe de réglage (39) qui est susceptible de modifier la force d'entraînement.

10. Instrument de percussion selon l'une des revendications 1 à 3, caractérisé par le fait que la section (19a) du levier oscillant (19), qui est éloignée de la tête d'examen (3) est constituée, au moins partiellement, avec un matériau magnétique doux et qu'un électro-aimant (27) est disposé dans le boîtier de l'instrument (17), de façon à correspondre à cette section (19a) que dans la zone du champ magnétique de l'électro-aimant (27) qui sert à l'entraînement, et de préférence à l'extrémité qui est éloignée de la tête d'examen (3), est disposé un aimant permanent (28) de manière que selon le sens du courant ($J_{sp}$) parcourant la bobine, un effet d'attraction ou de répulsion soit produit.

11. Instrument de percussion selon l'une des revendications 1 à 10, caractérisé par le fait qu'au point de sortie de la tête d'examen (3) il est prévu dans le boîtier de l'intrument (17) un dispositif d'éclairage (11) qui produit une tache lumineuse fortement concentrée qui, dans des plans perpendiculaires à la direction du déplacement de la tête d'examen (3), possède la forme d'un cercle.

12. Instrument de percussion selon l'une des revendications 1 à 11, caractérisé par le fait que le levier oscillant (19) est arrêté par une butée (26) dans la phase d'accélération avant d'atteindre l'objet, et que le signal produit de ce fait dans le capteur d'accélération (20) est appliqué à un amplificateur (31) pour ensuite être comparé dans un comparateur de valeur de consigne-valeur réelle, la réalisation étant telle que lors d'un écart par rapport à la valeur de consigne, un signal optique ou acoustique est produit.

13. Instrument de percussion selon la revendication 12, caractérisé par le fait qu'une lampe d'affichage (9) est disposée sur l'instrument.

14. Instrument de percussion selon l'une des revendications 1 à 13, caractérisé par le fait que l'on prévoit, en tant que capteur d'accélération un ou plusieurs éléments piézocéramiques, connus en eux-mêmes, lesquels sont disposés dans la partie antérieure de la section (19b) du levier oscillant (19), qui reçoit la tête d'examen (3), de préférence dans la tête d'examen (3) elle-même, de façon que dans le cas d'un rebondissement de la tête d'examen (3) sur l'objet à examiner, une grande partie de la masse du levier oscillant (19) agisse comme force d'inertie sur les éléments (22).

FIG 1

FIG 2

FIG 3

FIG 4

FIG 5

FIG 6